# EUROPÄISCHE PATENTANMELDUNG

(11) **EP 0 646 620 A1**
(43) Veröffentlichungstag der Anmeldung: **05.04.1995**
(21) Anmeldenummer: 94114771.2
(22) Anmeldetag: 20.09.1994
(51) Int. Cl.: C08J 11/04, C12P 5/02

(54) **Verfahren zur Verwertung von Reststoffen enthaltend cellulosische Fasern und Fasern aus synthetischen Polymeren**

(30) Priorität: 01.10.1993 DE 4333547
(71) Anmelder: HOECHST AKTIENGESELLSCHAFT, D-65929 Frankfurt am Main (DE)
(72) Erfinder: Huber, Bernd, Dr., D-93309 Kelheim (DE); Stein, Gerhard, D-93309 Kelheim (DE)

(57) **Zusammenfassung**

Beschrieben wird ein Verfahren zur Verwertung von Reststoffen aus cellulosefaserhaltigen Fasermischungen umfassend die Schritte:
i) Einsatz von Fasermischungen enthaltend cellulosische Fasern und Fasern aus synthetischen Polymeren, und
ii) Unterwerfen dieser Mischungen einer mikrobiellen Hydrolyse, bei der die cellulosischen Fasern vollständig abgebaut werden.

Mit dem Verfahren ist insbesondere die Auftrennung von Fasermischungen möglich. Dazu werden nach Schritt ii) die Mikroorganismen und das Hydrolysat in an sich bekannter Weise entfernt, und die verbleibenden synthetischen Polymeren in an sich bekannter Weise einer Wiederverwertung zugeführt.

## Beschreibung

Die Erfindung betrifft ein Verfahren zur Verwertung von Reststoffen enthaltend Gemische aus cellulosischen Fasern und Fasern aus synthetischen Polymeren.

Zur Vermeidung steigender Abfallmengen und zur besseren Nutzung der Ressourcen ist ein Recycling von Fasermaterialien, auch auf dem textilen Sektor, in immer größeren Maßen erforderlich.

Dabei ist eine Wiederaufarbeitung durch Zerreißen von Fasermaterialien und weitgehende Überführung in Einzelfasern in Form von sogenannter Reißwolle schon lange bekannt und üblich, führt im allgemeinen aber nur zu qualitativ anspruchslosen oder qualitativ minderwertigen Artikeln.

Recycling Verfahren, bei denen die Ketten synthetischer Polymerer in die Monomere zerlegt und dann für eine Neusynthese zur Verfügung stehen, vermeiden diese Nachteile und führen zu qualitativ hochwertigen Produkten.

Solche Verfahren sind z.B. für Polyesterfasern entwickelt, wie beispielsweise in der DE-A-2,506,259 beschrieben, und werden angewandt. Sie lassen sich allerdings nur bei sortenreinen Abfällen anwenden.

Große Mengen von Synthesefasern, insbesondere Polyesterfasern, werden zusammen mit cellulosischen Fasern, wie Baumwolle, Viskosefasern oder neu entwickelten aus Lösung ersponnenen cellulosischen Fasern, wie z.B TENCEL®, in Mischungen verarbeitet.

Eine chemische Abtrennung von cellulosischen Fasern mit starken Säuren als Lösungsmittel ist möglich, erfordert aber einen hohen Chemikalieneinsatz und belastet die Umwelt.

Ferner ist bekannt, Bioabfälle, wie Hausmüll oder andere kompostierbare Abfälle, einer mikrobiellen Hydrolyse zu unterwerfen und das Hydrolysat z.B. durch anaerobe Vergärung weiterzuverwerten.

Dabei gelingt es, die biogenen organischen Substanzen zum größten Teil, beispielsweise zu 65 - 75 %, zu hydrolysieren und das Hydrolysat in gasförmige und brennbare Bestandteile, die hauptsächlich aus Methan bestehen (sogenanntes Biogas), zu überführen.

Für Celluloseregeneratfaser ist es bekannt, daß der hydrolytische Abbau im Vergleich zu nativer Cellulose rascher und vollständiger verläuft (vergl. I. Schnurz; A. Hönel: Enzymatische Hydrolyse von regenerierten Cellulosefasern mit Cellulose aus Trichoderma; Cellulose Chemistry And Technology, 23, 466-476 (1989)).

Die Aufarbeitung von Mischungen von Cellulosefasern und vollsynthetischen Fasern ist noch nicht beschrieben worden.

Es bestand also die Aufgabe, ein einfaches Verfahren zur Auftrennung und Verwertung von Fasermischungen bereitzustellen, die aus cellulosischen Fasern und Fasern aus synthetischen Polymeren bestehen.

Es wurde gefunden, daß sich der hydrolytische Abbau von cellulosischen Fasern vorteilhaft für die Auftrennung und Aufarbeitung von Fasermischungen aus cellulosischen und vollsynthetischen Fasern eignet. Dabei werden die cellulosischen Fasern vollständig abgebaut und das vollsynthtischen Fasermaterial kann nach an sich bekannten Methoden recyclet werden, nachdem die Mikroorganismen entfernt worden sind, beispielsweise über eine Wäsche mit Wasser. Der Energieinhalt des Hdrolysates kann vorteilhaft über eine anaerobe Vergärung in Biogas übergeführt werden.

Die Erfindung betrifft ein Verfahren zur Verwertung von Reststoffen aus cellulosefaserhaltigen Fasermischungen umfassend die Schritte:
i) Einsatz von Fasermischungen enthaltend cellulosische Fasern und Fasern aus synthetischen Polymeren, und
ii) Unterwerfen dieser Mischungen einer mikrobiellen Hydrolyse, bei der die cellulosischen Fasern vollständig abgebaut werden.

In einer besonders bevorzugten Ausführungsform eignet sich das Verfahren auch zu einer Stofftrennung von Fasermischungen aus vollsynthetischen Fasern und cellulosischen Fasern.

In dieser Ausführungsform des erfindungsgemäßen Verfahrens werden nach Schritt ii) die Mikroorganismen und das Hydrolysat in an sich bekannter Weise entfernt, beispielsweise über eine Wäsche mit Wasser, und die verbleibenden synthetischen Polymeren werden in an sich bekannter Weise einer Wiederverwertung zugeführt, sei es durch Verwertung dieser synthetischen Polymeren durch Verbrennung oder durch Wiedereinsatz oder durch Zerlegung in Monomere.

In einer bevorzugten Ausführungsform des erfindungsgemäßen Verfahrens werden Mischungen eingesetzt, die a) Fasermischungen enthaltend cellulosische Fasern und Fasern aus synthetischen Polymeren und b) weitere mikrobiell hydrolisierbare Bestandteile enthalten.

Hinsichtlich des Polymertyps der Fasern aus synthetischen Polymeren ist das erfindungsgemäße Verfahren keinen Einschränkungen unterworfen.

Die Reststoffe können in Form von Garnen oder auch als textile Flächengebilde, wie Gewirke, Gestricke, Gewebe, Vliese oder Filze, eingesetzt werden. Hinsichtlich des Titers und der Faserlänge ist das erfindungsgemäße Verfahren keinen Einschränkungen unterworfen. Es lassen sich Materialien aus Stapelfasern oder auch aus Endlos-Filamenten einsetzen.

Bevorzugte Substrate für cellulosische Fasern sind beispielsweise: Baumwolle, Viskose, Modal und aus Lösungsmittel ersponnene cellulosische Fasern.

Bevorzugte Substrate für Fasern aus synthetischen Polymeren sind beispielsweise: Polyester, beispielsweise Polyethylen- oder Polybutylenterephthalate, Polyamide, wie Perlon- oder Nylon-Typen, Polyolefine, beispielsweise Polypropylen oder Polyethylen, oder Aramide.

Das erfindungsgemäße Verfahren kann in großtechnischen Anlagen zur Verwertung von Biomüll, wie Gartenabfällen oder Küchenabfällen, durchgeführt werden. Für die technische Durchführung in diesen für Biomüll optimierten Anlagen ist eine vorherige Zerkleinerung durch Zerschneiden oder Zerreißen von Garnen oder Flächengebilden aus Fasermischungen vorteilhaft, um das Fördern der Suspension in wäßriger Aufschlämmung zu gewährleisten.

Das erfindungsgemäße Verfahren kann in Gegenwart von Biomüllsuspensionen oder in Reinform in wässriger Aufschlämmung durchgeführt werden. Werden nur Fasermischungen eingesetzt, so empfiehlt es sich, für das Wachstum der Hydrolase produzierenen Bakterien die erforderlichen Spurenelemente zuzusetzen.

Um nach der Hydrolyse der cellulosischen Fasern die Reinigung der zurückgebliebenen Fasern aus synthetischen Polymeren zu erleichtern, ist es vorteilhaft, den Anteil der Biomüllsuspension auf den für den Prozeß erforderlichen Mindestgehalt zu beschränken oder ganz darauf zu verzichten.

Ganz besonders bevorzugt wird nach der mikrobiellen Hydrolyse eine anaerobe Vergärung durchgeführt, wobei das Hydrolysat vollständig zu Biogas vergoren wird. Die anaerobe Vergärung des Hydrolysates kann auch zusammen mit der mikrobiellen Hydrolyse in einem Schritt erfolgen.

Das folgende Beispiel beschreibt die Erfindung ohne diese zu begrenzen.

### Beispiel 1:

### Hydrolyse einer Polyester-Viskosefasermischung

Ein Mischgarn aus Polyester/Viskose im Verhältnis 65/35 % wurde einem kombinierten Hydrolyse- und Gärtest unterzogen. Zusammen mit dem Mischgarn wurde eine Biomüllsuspension aus Gartenabfällen und nassem Biomüll aus einer technischen Anlage für Biomüllverwertung (Gehalt von 10 g organischer Trockenmasse) eingesetzt. Die Ansätze wurden in einem Einstufenverfahren hydrolysiert und direkt anaerob vergoren.

Zur Hydrolyse wurden zu 100 ml der Biomüllsuspension 3,5 g eines Polyester/Viskose-Mischgarnes aus 65 % Polyesterfasern mit einem Titer von 1,7 dtex und 35 % einer Viskosefaser mit einem Titer von 1,3 dtex zugesetzt.

Nach Versuchsende nach 300 h wurde das Restgarn, welches in seiner Garnstruktur erhalten blieb, mit heißem Wasser gewaschen und anschließend getrocknet.

Über mikrospische und chemische Prüfungen konnten keine Anteile an Viskosefasern gefunden werden.

Der Restanteil bestand, wie die Analyse zeigte, aus reinen Polyesterfasern, die leicht recyclet werden können.

## Patentansprüche

1. Verfahren zur Verwertung von Reststoffen aus cellulosefaserhaltigen Fasermischungen umfassend die Schritte:
i) Einsatz von Fasermischungen enthaltend cellulosische Fasern und Fasern aus synthetischen Polymeren, und
ii) Unterwerfen dieser Mischungen einer mikrobiellen Hydrolyse, bei der die cellulosischen Fasern vollständig abgebaut werden.

2. Verfahren nach Anspruch 1, dadurch gekennzeichnet, daß nach Schritt ii) die Mikroorganismen und das Hydrolysat in an sich bekannter Weise entfernt, und die verbleibenden synthetischen Polymeren in an sich bekannter Weise einer Wiederverwertung zugeführt werden.

3. Verfahren nach Anspruch 1, dadurch gekennzeichnet, daß Mischungen eingesetzt werden, die a) Fasermischungen enthaltend cellulosische Fasern und Fasern aus synthetischen Polymeren und b) weitere mikrobiell hydrolisierbare Bestandteile enthalten.

4. Verfahren nach Anspruch 1, dadurch gekennzeichnet, daß die Fasern aus synthetischen Polymeren Polyesterfasern sind.

5. Verfahren nach Anspruch 1, dadurch gekennzeichnet, daß im Anschluß an die mikrobielle Hydrolyse das Hydrolysat anaerob zu Biogas vergoren wird.

6. Verfahren nach Anspruch 5, dadurch gekennzeichnet, daß die mikrobielle Hydrolyse und die anaerobe Vergärung in einem Verfahrensschritt gemeinsam durchgeführt werden.
